(19) Europäisches Patentamt
European Patent Office
Office européen des brevets

(11) Publication number: 0 186 409
A2

(12) EUROPEAN PATENT APPLICATION

(21) Application number: 85309166.8

(22) Date of filing: 16.12.85

(51) Int. Cl.[4]: B 01 J 31/40

(30) Priority: 21.12.84 US 685097

(43) Date of publication of application:
02.07.86 Bulletin 86/27

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Forman, Andrew L.
999 HiddenLake Drive Apt. 3B
North Brunswick New Jersey 08902(US)

(74) Representative: Crampton, Keith John Allen et al,
D YOUNG & CO 10 Staple Inn
London WC1V 7RD(GB)

(54) **Homogeneous catalyst recovery.**

(57) A process for recovering a spent homogeneous catalyst from a reaction mixture comprises:

(1) contacting the mixture with a resin bearing a non-ionic heterocyclic electron donor residue;
(2) filtering off the resin after step (1);
(3) freeing the homogeneous catalyst complex from the resin obtained in (2) by displacement with a suitable ligand; and
(4) recovering the homogeneous catalyst from step (3) in its active form.

EP 0 186 409 A2

# HOMOGENEOUS CATALYST RECOVERY

The present invention relates to the recovery of spent homogeneous metallic catalyst.

Homogeneous catalysis is used in a number of commercially useful processes, e.g. hydroformylation of olefins. These catalysts are commonly salts and/or complexes of a Group VIII metal such as cobalt, rhodium, ruthenium and iridium. While it is desirable to recover catalysts in general, it is especially important to recover catalysts which contain exotic and more expensive metals such as rhodium and iridium. It is also advantageous to recover the catalyst in an active form substantially ready for direct re-use or recycle.

A process has now been discovered by which homogeneous metallic catalysts may be recovered in an active form suitable for direct re-use.

In accordance with the present invention, a process for recovering a spent homogeneous catalyst from a reaction mixture comprises:

(1) contacting the mixture with a resin bearing a non-ionic heterocyclic electron donor residue;

(2) filtering off the resin after step (1);

(3) freeing the homogeneous catalyst complex from the resin obtained in (2) by displacement with a suitable ligand; and

(4) recovering the homogeneous catalyst from step (3) in its active form.

The homogeneous catalyst is a metal-containing compound or complex in which the metal is of Group VIII, particularly the platinum group, preferably rhodium and especially rhodium (II). Examples of the metal catalyst compounds/complexes are the metal $C_{2-10}$ carboxylates and the metal complexes with ligands such as CO; $R_3Z$, $(RO)_3Z$, and $(RO_2)ZR$ where R is a suitable alkyl, aryl, aralkyl, or alkaryl group and Z is P, Sb or As, preferably P. Catalysts of special interest are rhodium carboxylates, preferably Rh(II) carboxylates, particularly Rh(II) $C_{2-8}$ carboxylates and especially Rh(II) octanoate dimer.

The resin used must have in its structure a suitable heterocyclic electron donor residue. The resin of course, is an insoluble organic polymer such as that obtained by polymerization of a suitable monomer or mixture of monomers and subsequent cross-linking as appropriate. Among suitable electron donor residues are heterocyclic groups such as a pyridine, imidazole and bis-picolamine. Suitable resins include cross-linked and linear polymers of 2-vinylpyridine, 4-vinylpyridine and vinylimidazole. An especially useful resin is a cross-linked vinyl pyridine resin.

The catalyst bound to the resin is separated or displaced from it by tretment with any suitable unattached ligand. Such ligands are illustrated by compounds such as dimethylformamide, $CH_3CN$, pyridine, tetrahydrofuran, methanol and dimethylsulfoxide, preferably $CH_3CN$.

Usually, the displacement is achieved by treating the resin bearing the bound catalyst with an organic acid to neutralize the electron donor (Lewis base) residue, followed by treatment with an unattached ligand alone or in a compatible solvent, such as a mixture of methylene chloride containing formic or acetic acid. The catalyst, in active form, is then recovered after displacement by conventional means such as vacuum distillation or extraction. The recovered catalyst is active and may then be recycled directly into the process in which it was originally spent.

The following scheme is provided to illustrate the present process. The catalyst recovered is Rh(II) alkanoate and the resin is pyridyl-bearing:

A.)

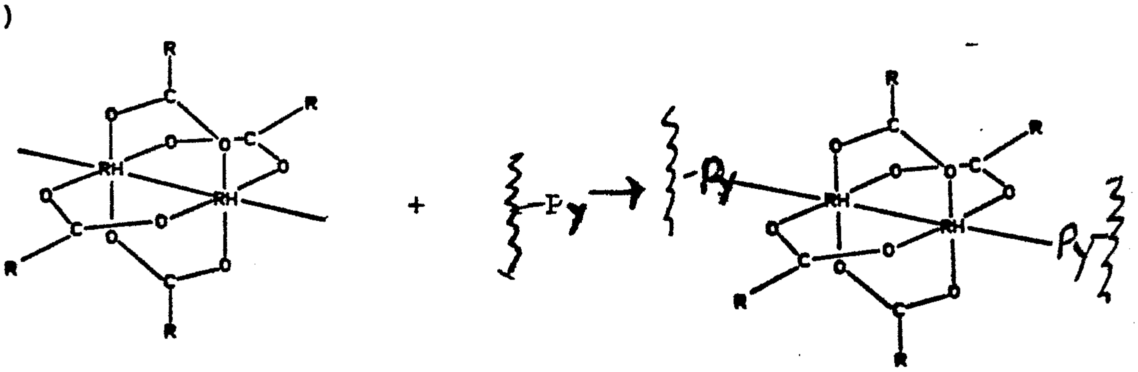

I (spent) II (resin) III

B.) III + Protonating Acid/ Ligand (L)/ Solvent

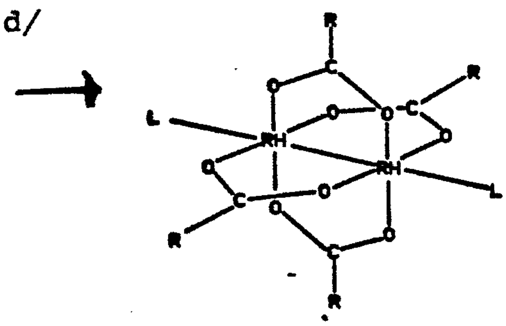

+ II $H^+$

IV

C.) IV $\xrightarrow{\Delta,\ vac.}$ I (active)

Py = pyridine

R = as defined above

An example of a catalytic process where homogenous catalyst recovery is of value is in the cyclization reaction illustrated by the following reaction equation:

EQUATION A

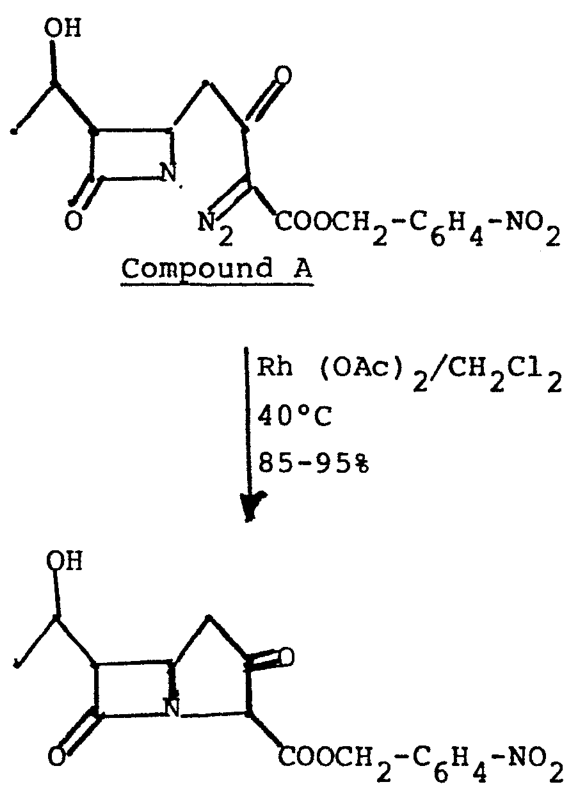


Compound A

Compound B

The intermediate B is useful in the preparation of the antibiotic thienamycin; the reaction is described in Journal of the American Chemical Society, 100, 313 (1978), (D. B. R. Johnston, S. M. Schmitt, F. A. Bouffard, and B. G. Christensen).

The following example illustrates the present catalyst recovery system. The reaction involved is that illustrated by Equation A.

EXAMPLE 1

FLOW SHEET

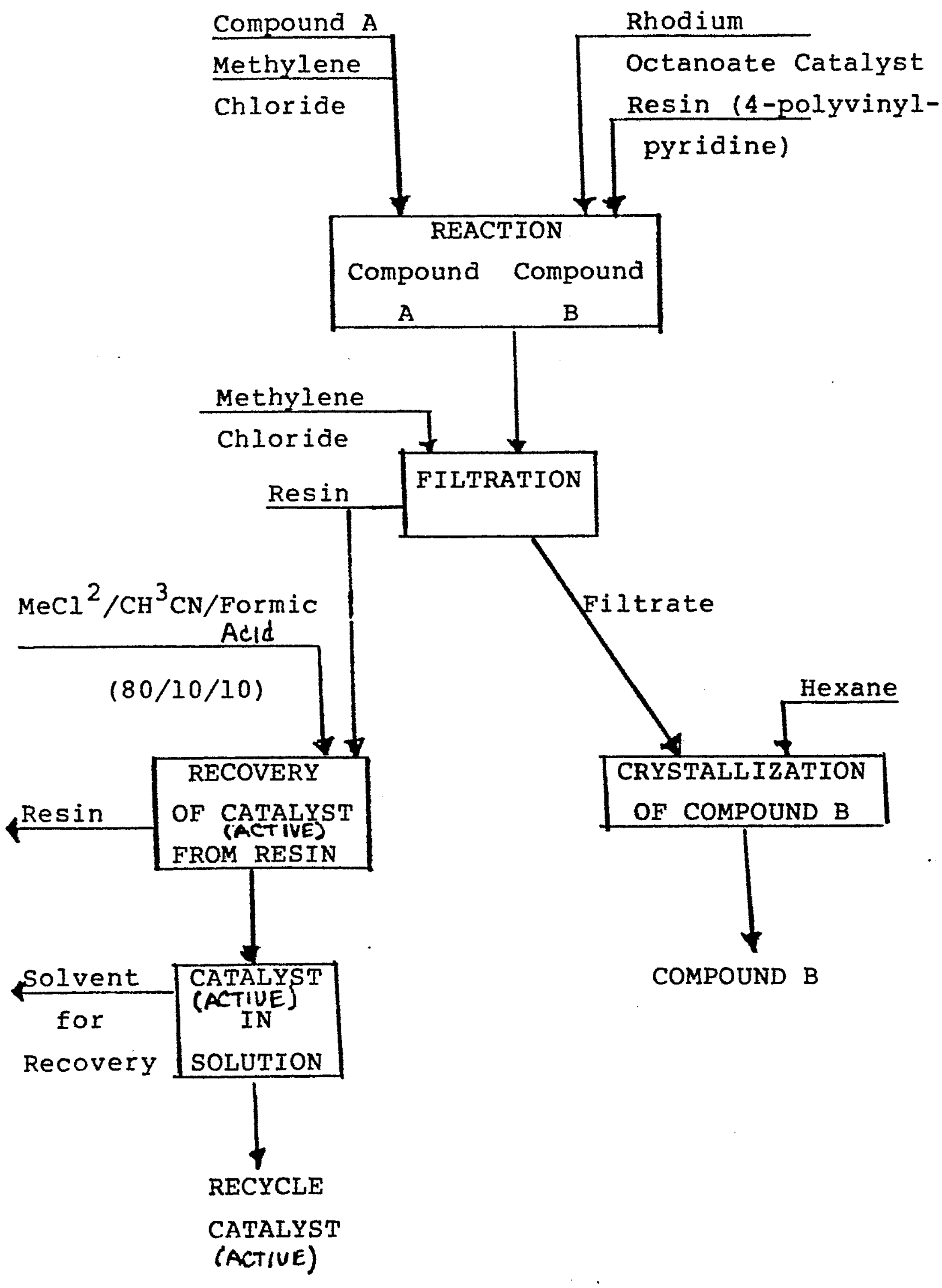

Compound A (250 gms) was azeotropically dried in methylene chloride (3 liters) at 40°C. When 500 ml of distillate were collected, the solution was cooled to room temperature. Then rhodium octanoate (1 gm) was added to the solution and it reacted with Compound A at 40°C to form a solution of Compound B. Eighty percent (by volume) of the homogenous solution was cooled and treated for two hours with the resin, poly-4-vinylpyridine cross-linked beads[1] (20 grams). The solution was filtered and the resin was washed with methylene chloride (400 ml) to ensure complete recovery of Compound B. The filtrate and wash was subdivided into 17/100 and 83/100 parts by volume. Compound B was crystallized from 83% by volume of the filtrate by slow addition of n-hexane (200 ml). The solution was aged at room temperature for 1 hour and at 5°C for 15 minutes. Then it was filtered and the cake washed with 1200 ml of 50/50 $MeCl_2$/n-hexane (cold). The yield and purity of Compound B were 85% and 96% respectively. The catalyst was recovered by treating the resin sequentially with 1500 ml of $MeCl_2$/acetonitrile/formic acid (80/10/10; v/v) and 500 ml of $MeCl_2$. The filtrate was concentrated on a rotary evaporator and the catalyst was deposited on the walls of the flask. The catalyst was dissolved in 250 ml of methylene chloride.

Assuming 75% recovery of active catalyst, the recovered catalyst was recycled. In the second cycle, Compound A (110 gm) was azeotropically dried in methylene chloride (1.8 liter) at 40°C. When 886 ml of distillate were collected, the solution was cooled to room temperature. Then the recovered catalyst solution (186.4 ml) was added to the batch

and it reacted with Compound A at 40°C to form a homogenous solution of Compound B. The homogenous solution was cooled and treated for two hours with 8.8 gm of resin, poly-4-vinylpyridine cross-linked beads[1]. The solution was filtered and the resin was washed with 200 ml of methylene chloride to ensure complete recovery of Compound B. Compound B was crystallized by slow addition of hexane (1300 ml) to the filtrate. The solution was aged at room temperature for 1 hour and at 5°C for 15 minutes. The solution was filtered and the cake washed with 800 ml of $MeCl_2$/n-hexane (cold). The yield and purity of Compound B were 96% and 97% respectively. The catalyst was recovered by treating the resin sequentially with 660 ml of $MeCl_2$/acetonitrile/ formic acid (80/10/10; v/v) and 400 ml of $MeCl_2$. The solution was filtered. The filtrate was concentrated on a rotary evaporator. The catalyst was deposited on the walls of the flask and was dissolved in 250 ml of methylene chloride.

Assuming 75% recovery of active catalyst, the recovered catalyst was recycled in the third cycle. In the third cycle, Compound A (75 gm) was azeotropically dried in methylene chloride (1250 ml) at 40°C. When 727 ml of distillate were collected, the solution was cooled to room temperature. Then the recovered catalyst solution (227 ml) was added to the batch and it reacted with Compound A at 40°C to form a homogenous solution of Compound B. The homogenous solution was cooled and treated for two

hours with 6 grams of poly-4-vinylpyridine cross-linked beads[1]. The solution was filtered and the resin was washed with 400 ml of methylene chloride to ensure recovery of Compound B. Compound B was crystallized by slow addition of n-hexane (1150 ml) to the filtrate. The solution was aged at room temperature for 1 hour and 5°C for 15 minutes. The solution was filtered and the cake washed in 544 ml of $MeCl_2$/n-hexane (cold). The yield and purity of Compound B were 97% and 97% respectively. The catalyst was recovered by treating the resin sequentially with 300 ml of $MeCl_2$/acetonitrile/formic acid (80/10/10; v/v) and 400 ml of $MeCl_2$. The solution was filtered. The filtrate was concentrated on a rotary evaporator. The catalyst was deposited on the walls of the flask and dissolved in 250 ml of methylene chloride.

While the Example illustrates recovery of the catalyst prior to crystallization of Compound B, the spent catalyst may also be recovered from the reaction mixture after crystallization of Compound B if desired. The example clearly illustrates that the catalyst recovered is active since it is directly recycled into the reactions illustrated by equation A. The recovery of catalyst in an active form offers

---

1 The resin, which was a commercial product (425X) obtained from Reilly Tar, was pretreated sequentially with methanol [12.5 ml/gm of resin (dry)] and methylene chloride [25 ml/gm of resin (dry)] to remove residual pyridine.

the opportunity of a continuous recycling of the catalyst, adding only make-up catalyst for that portion mechanically lost in the processing and handling steps.

Other processes using homogenous catalysts to which the present process is also applicable are listed below:

i) For the cyclopropanation of 1,1-dihalo-4-methyl-penta-1,3-dienes with ethyl diazoacetate.

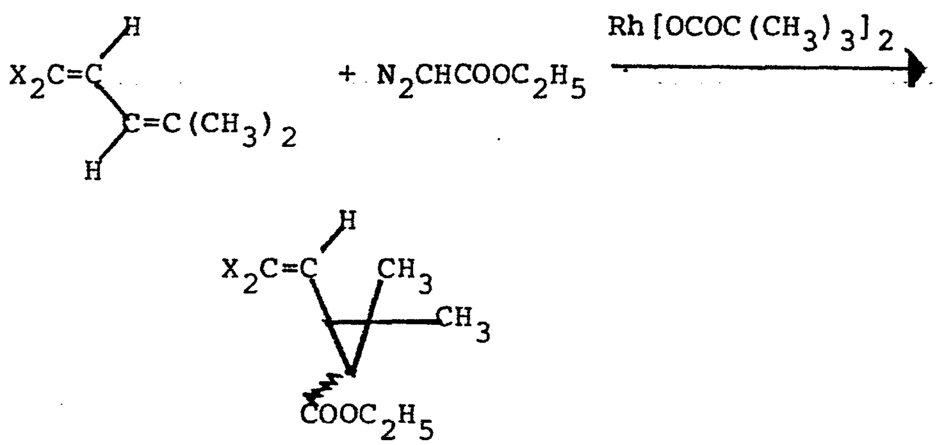


ii) For the cyclization of diazo compounds to ß-lactams.

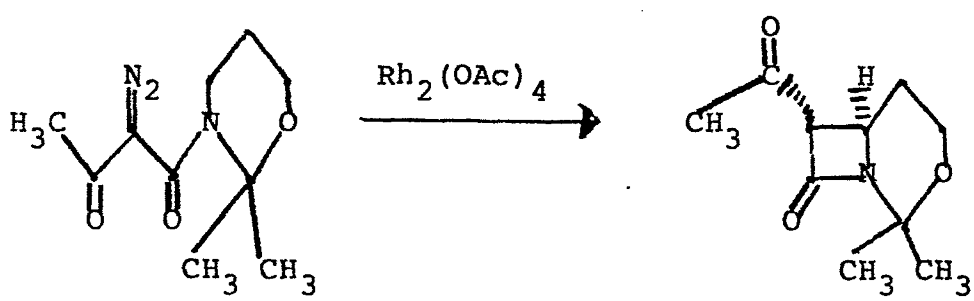


iii) For the rearrangement of α-diazo-ß-hydroxy-,8-unsaturated esters to ß-keto esters.

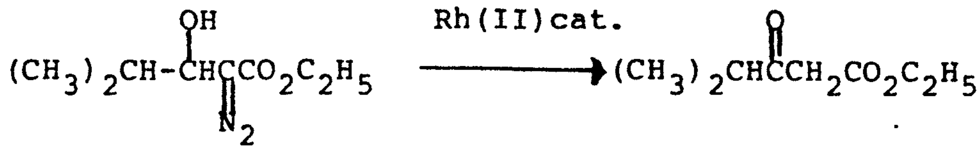


iv) Hydroformylation using a conventional homogenous rhodium, iridium, cobalt or ruthenium catalyst.

Using procedures substantially like that of Example 1, the spent catalyst from reactions such as i), ii), iii) and iv) can be recovered in active form for recycle to the respective processes.

## CLAIMS

1. A process for recovering a spent homogeneous catalyst from a reaction mixture that comprises:

   (1) contacting the mixture with a resin bearing a non-ionic heterocyclic electron donor residue;
   (2) filtering off the resin after step (1);
   (3) freeing the homogeneous catalyst complex from the resin obtained in (2) by displacement with a suitable ligand; and
   (4) recovering the homogeneous catalyst from step (3) in its active form.

2. A process as claimed in claim 1 in which the catalyst contains a Group VIII metal.

3. A process as claimed in claim 2 in which the metal is rhodium.

4. A process as claimed in claim 3 in which the catalyst is a rhodium (II) $C_2$-$C_8$ alkanoate.

5. A process as claimed in any preceding claim in which the heterocyclic electron donor residue is a pyridine compound.

6. A process as claimed in claim 5 in which the pyridine compound is pyridine.

7. A process as claimed in claim 6 in which the ligand is $CH_3CN$.

8. A process as claimed in claim 6 in which the ligand is THF or methanol.

9. A process as claimed in claim 7 in which the catalyst is rhodium octanoate.

10. A homogeneous catalyst recovered from step (4) in any one of the preceding claims.